# EUROPÄISCHE PATENTSCHRIFT

(11) **EP 3 106 194 B1**
(45) Veröffentlichungstag und Bekanntmachung des Hinweises auf die Patenterteilung: **13.03.2019**
(21) Anmeldenummer: 15172173.5
(22) Anmeldetag: 15.06.2015
(51) Int. Cl.: A61M 5/32

(54) **SICHERHEITSVORRICHTUNG FÜR EINE SPRITZE**
SAFETY DEVICE FOR A SYRINGE
DISPOSITIF DE SÉCURITÉ POUR UNE SERINGUE

(43) Veröffentlichungstag der Anmeldung: 21.12.2016
(60) Teilanmeldung: 18168709.6
(73) Patentinhaber: Gerresheimer Regensburg GmbH, 93047 Regensburg (DE); Gerresheimer Bünde GmbH, 32257 Bünde (DE)
(72) Erfinder: Wittland, Frank, 32257 Bünde (DE); Vogl, Maximilian, 92708 Mantel (DE)
(74) Vertreter: Hannke, Christian

(56) Entgegenhaltungen:
- WO-A1-01/54758
- WO-A1-2012/096620
- US-A- 5 591 138
- US-A1- 2014 257 200

## Beschreibung

Die Erfindung betrifft eine Sicherheitsvorrichtung zur Vermeidung von Stichverletzungen für eine Spritze mit einem Spritzenkörper und einem an dem distalen Ende des Spritzenkörpers angeordneten Stechmittel, umfassend unter anderem ein sich entlang einer axialen Richtung (X) erstreckendes Hülsenelement, welches zumindest teilweise das Stechmittel und den Spritzenkörper umschließt, und ein Kragenelement, welches an einem distalen Endbereich des Spritzenkörpers anordenbar ist und die Sicherheitsvorrichtung in axialer Richtung (X) arretiert, wobei das Kragenelement zumindest einen Führungsvorsprung aufweist, welcher in zumindest einer Führungskulisse des Hülsenelements eingreift.

Gattungsgemäße Sicherheitsvorrichtungen zur Vermeidung von Stichverletzungen sind aus dem Stand der Technik bekannt. Insbesondere bei vorgefüllten Spritzen ist die Verwendung derartiger Sicherheitsvorrichtungen sinnvoll. Die Handhabung derartiger Spritzen ist sehr einfach, da das Medium nicht vor der Anwendung in die Spritze transferiert werden muss. Weiterhin ist, selbst im Notfall, die Wahrscheinlichkeit der Anwendung eines falschen Medikaments sehr gering. Für Impfstoffe und zahlreiche andere Medikamente sind sie heutzutage das Primärpackmittel erster Wahl. Diese Spritzen sind üblicherweise aus Glas oder Kunststoff (beispielsweise COC, COP) hergestellt und müssen mit Schutzkappen ausgestattet sein, um Beschädigungen und/oder eine Kontaminierung der Kanüle vor der Anwendung der Spritze zu vermeiden. Darüber hinaus ist es wichtig, nach Gebrauch der Spritze die Kanüle zu sichern, um Stichverletzungen zu vermeiden. Dabei kann ein unvorsichtiges Wiederaufsetzten der Schutzkappe auf die Kanüle Stichverletzungen verursachen. Oft ist die entsprechende Schutzkappe nicht mehr auffindbar oder es wird vergessen, diese wiederaufzusetzen, wodurch ein vermeidbares Verletzungsrisiko gegeben ist.

Demzufolge wurden Nadelschutzeinrichtungen entwickelt, welche fest mit der Spritze verbunden sind und die Nadel nach Verwendung der Spritze automatisch wieder aufnehmen. Eine solche Nadelschutzeinrichtung ist beispielsweise in DE 11 2009 001 083 T5 offenbart. Hierbei wird eine federangetriebene Sicherheitshülse gezeigt, welche in einem ausgefahrenen Zustand die Kanüle umgibt und diese gegen Verletzungen der Anwender sichert. Die Sicherheitshülse weist dabei eine Kurvenbahn auf, in welcher mindestens ein Führungsstift läuft, wodurch verschiedene Positionen der Sicherheitshülse in Abhängigkeit zur Nadelspitze realisiert werden können. Der mindestens eine Führungsstift muss dabei über einen Kragen an der Frontgeometrie der Spritze befestigt werden oder muss auf andere Weise fest mit der Spritze verbunden werden. Der Kragen mit dem Führungsstift darf, um Manipulation oder Fehlbenutzung vorzubeugen, nicht oder nur schwer von der Spritze mit einer Kanüle entfernbar sein. Demnach ist ein entsprechend fester Sitz in axialer Richtung notwendig.

Im Bereich der vorgefüllten Spritzen wird bereits vor dem Abfüllprozess eine Schutzkappe beziehungsweise eine Sicherheitsvorrichtung zur Vermeidung von Stichverletzungen auf dem Spritzenkörper montiert und in einer Standardverpackung beispielsweise einem Spritzennest sterilisiert wird. Man spricht in diesem Zusammenhang auch von Ready to Use (RTU) bzw. Ready to Sterilize (RTS) Spritzen. In der Regel sollen die Sicherheitsvorrichtungen derart ausgelegt sein, dass sie leichtgängig zu betätigen sind, um einen optimalen Komfort für den Anwender zu gewährleisten. Demzufolge ist es möglich, dass die Spritzen bereits während des Transports ungewollt betätigt werden. Auch können die Spritzen versehentlich während der Benutzung ausgelöst werden. Dies kann die Spritze unter Umständen komplett unbrauchbar machen. Das beinhaltete Medikament kann nicht verabreicht werden. Überdies kann es ebenso zu Verletzungen des Anwenders oder des Patienten kommen, falls dieser die Sicherheitsvorrichtung unabsichtlich Eine gattungsgemäße Sicherheitsvorrichtung ist auch aus US20124/0257200 A1 bekannt. Dieses Dokument offenbart eine Sicherheitsvorrichtung, welche aus einem fest montierten Kragenelement mit zwei Hülsenelementen und einem Kappenelement besteht. Der Kragen dichtet die Spritze gegenüber der Umgebung ab.

Aufgabe der vorliegenden Erfindung ist es, eine Sicherheitsvorrichtung zur Vermeidung von Stichverletzungen für eine Spritze zur Verfügung zu stellen, welche die eingangs genannten Probleme löst.

Diese Aufgabe wird gelöst durch eine Sicherheitsvorrichtung mit den Merkmalen von Anspruch 1.

Da durch das Kappenelement das Hülsenelement bezüglich der Relativbewegung des Spritzenkörpers zu dem Hülsenelement arretierbar ist, wird effektiv ein unerwünschtes Hervortreten des Stechmittels, welches eine Kanüle, eine Nadel oder auch eine Lanzette sein kann, durch eine entsprechende Öffnung in der Sicherheitsvorrichtung unterbunden. Beschädigungen und Kontamination des Stechmittels werden demnach verhindert. Der Anwender der Spritze muss zunächst das Kappenelement von dem Hülsenelement abziehen, bevor die Spritze angewendet werden kann. Demnach wird ebenso das Risiko eines versehentlichen Betätigens verringert. Es wäre denkbar, an dem Kappenelement eine Markierung oder einen Hinweis anzubringen. Der Anwender wäre somit gezwungen, diese Markierung beziehungsweise den Hinweis vor der Anwendung der Spritze wahrzunehmen. Eine derartige Markierung beziehungsweise ein derartiger Hinweis könnte farblich und/oder haptisch und/oder anderweitig ausgestaltet sein.

Gemäß einer besonders bevorzugten Ausführungsform ist das Kragenelement im Wesentlichen als hohler Kreiszylinder ausgebildet. Bevorzugt weist der Kreiszylinder eine Mantelfläche auf, an der der zumindest eine Führungsvorsprung angeordnet ist. Vorzugsweise erstreckt sich der zumindest eine Führungsvorsprung radial von der Mantelfläche weg. Weiterhin bevorzugt ist der Führungsvorsprung als Kreiszylinder beziehungsweise als Stift ausgebildet. Vorteilhafterweise sind an der Mantelfläche zwei sich diametral gegenüberliegende Führungsvorsprünge angeordnet. Demzufolge würde auch das Hülsenelement zwei sich diametral gegenüberliegende Führungskulissen aufweisen, in denen jeweils ein Führungsvorsprung geführt wird. Erfindungsgemäß ist das Kragenelement weiterhin in einer Umfangsrichtung (U) rotierbar an dem distalen Endbereich des Spritzenkörpers angeordnet. Bei einer Anwendung der Spritze wird die Spritze mit der Sicherheitseinrichtung gegen die Haut des Patienten gedrückt. Durch die Relativbewegung des Spritzenkörpers zu dem Hülsenelement und die Führung des Führungsvorsprungs in der Führungskulisse wird eine Rotation des Kragenelements entlang einer Umfangsrichtung (U) verursacht. Das Hülsenelement schiebt sich dadurch über den Spritzenkörper, wodurch das Stechmittel, welches eine Kanüle, eine Nadel oder auch eine Lanzette sein kann, durch eine entsprechende Öffnung in dem Hülsenelement hindurchtritt. Somit wird eine Rotation des Hülsenelements auf der Haut des Patienten um die Einstichstelle herum vermieden.

Vorzugsweise ist der Spritzenkörper als hohler Kreiszylinder ausgestaltet und weist in seinem distalen Endbereich ein konisches Endstück auf, an welchem das Stechmittel angeordnet ist. Bevorzugt ist an dem konischen Endstück ein Vorsprung ausgebildet, an welchem eine Stirnfläche des distalen Endes des Kragenelements eingreifbar ist, wodurch das Kragenelement und somit die Sicherheitsvorrichtung in axialer Richtung arretierbar ist. Weiterhin bevorzugt ist auch die Sicherheitsvorrichtung im Wesentlichen in Form eines hohlen Kreiszylinders ausgestaltet.

Gemäß der Erfindung weist das Kappenelement eine Stechmittelschutzeinrichtung auf, in welcher das Stechmittel anordenbar ist. Durch eine derartige Stechmittelschutzeinrichtung wird ein weiterer Schutz des Stechmittels vor Beschädigungen und insbesondere vor Kontamination gewährleistet.

Vorzugsweise weist das Hülsenelement eine distale Öffnung auf. Bevorzugt ist dabei der Innendurchmesser der distalen Öffnung zumindest abschnittsweise größer als der Außendurchmesser der Stechmittelschutzeinrichtung, so dass die Stechmittelschutzeinrichtung innerhalb des Hülsenelements anordenbar ist.

Erfindungsgemäß ist die Stechmittelschutzeinrichtung mit dem Kragenelement in Wirkkontakt bringbar, wodurch das Kragenelement bezüglich einer Rotation arretierbar ist. Ein solcher Wirkkontakt kann beispielweise ein reibschlüssiger Kontakt sein. Es wäre aber auch denkbar, dass die Stechmittelschutzeinrichtung und das Kragenelement zueinander korrespondierende Rasteinrichtungen aufweisen, welche eine Rotation des Kragenelements verhindern.

Gemäß einer bevorzugten Ausführungsform weisen das Kappenelement und das Hülsenelement zueinander komplementäre Rastelemente auf, so dass das Kappenelement und das Hülsenelement trennbar verrastbar sind. Es wäre vorstellbar, dass ein Rastelement eine Sollbruchstelle aufweist, welche vor der Anwendung aufgebrochen werden muss, um ein Abziehen des Kappenelements von dem Hülsenelement zu ermöglichen. Es wäre aber auch denkbar, dass ein Einrasten der Rastelemente auch nach der Anwendung der Spritze ermöglicht ist. Somit wäre es ermöglicht, dass Kappenelement nach dem Gebrauch der Spritze wieder fest auf dem Hülsenelement anzuordnen, wodurch das Hülsenelement bezüglich der Relativbewegung des Spritzenkörpers zu dem Hülsenelement wiederum arretierbar wäre. Demzufolge ist eine gefahrlose Entsorgung der gebrauchten Spritze möglich, welche somit kein Verletzungsrisiko mehr darstellt.

Gemäß eines weiteren bevorzugten Gedankens der Erfindung umfasst das Kappenelement zumindest ein flügelartiges Element, welches in zumindest einer Aufnahme des Hülsenelements aufnehmbar ist. Weiterhin bevorzugt weist das Kappenelement zwei flügelartige Elemente auf, welche diametral in jeweils zwei Aufnahmen des Hülsenelements aufnehmbar sind. Besonders bevorzugt sind die beiden flügelartigen Elemente diametral sich gegenüberliegend an dem Kappenelement angeordnet.

Nach einer weiteren bevorzugten Ausführungsform ist zumindest ein flügelartiges Element mit dem Kragenelement in Wirkkontakt bringbar, wodurch das Kragenelement bezüglich einer Rotation arretierbar ist. Ein solcher Wirkkontakt kann beispielweise ein reibschlüssiger Kontakt sein. Es wäre aber auch denkbar, dass die Stechmittelschutzeinrichtung und das Kragenelement zueinander korrespondierende Rasteinrichtungen aufweisen, welche eine Rotation des Kragenelements verhindern.

Gemäß einer weiteren bevorzugten Ausführungsform ist an dem zumindest einen flügelartigen Element des Kappenelements zumindest ein Rastelement angeordnet, welches in zumindest ein komplementäres Rastelement, das in der zumindest einen Aufnahme des Hülsenelements angeordnet ist, einrastbar ist.

Es wär auch denkbar, dass das Hülsenelement einen distalen Bereich aufweist, an welchem zumindest ein Rastelement angeordnet ist. Vorteilhafterweise ist das zumindest eine Rastelement in zumindest ein komplementäres Rastelement einrastbar, welches in einem distalen Bereich des Kappenelements angeordnet ist.

Vorzugsweise ist das Kappenelement integral mit der Stechmittelschutzeinrichtung ausgebildet. Eine derartige Ausführung der Sicherheitsvorrichtung hat eine kostengünstige und einfache Herstellung zum Vorteil.

Es ist aber auch denkbar, dass das Kappenelement eine distale Öffnung aufweist, wobei die distale Öffnung als Aufnahme ausgebildet ist, um die Stechmittelschutzeinrichtung aufzunehmen. Eine solche Ausgestaltung ermöglicht es, das Kappenelement und die Stechmittelschutzeinrichtung aus unterschiedlichen Materialien herzustellen. Es wäre demnach denkbar, die Stechmittelschutzeinrichtung aus einem elastischen Material, beispielsweise Gummi herzustellen. Ein solches elastische Material begünstigt eine Reduzierung des Beschädigungsrisikos des Stechmittels.

Vorzugsweise weist die Sicherheitsvorrichtung mindestens ein Federelement auf, das mit dem Spritzenkörper wirkverbunden ist und der Relativbewegung des Spritzenkörpers zu der Sicherheitsvorrichtung entgegenwirkt. Demnach bleibt die Kanüle bis zur vorgesehenen Anwendung innerhalb des Hülsenelements. Bei der Anwendung muss das Hülsenelement gegen die Federkraft verschoben werden, damit die Kanüle durch die Öffnung des Hülsenelements hindurchtreten kann. Nach Gebrauch der Spritze schiebt sich automatisch, angetrieben durch die Federkraft des Federelements, das Hülsenelement wieder über die Kanüle. Durch die Führung des Führungsvorsprungs in der Führungskulisse rotiert das Kragenelement entgegen der Umfangsrichtung (U). Der Anwender ist somit vor Stichverletzungen mit der gebrauchten kontaminierten Kanüle geschützt. Bevorzugt umfasst das Federelement eine Spiralfeder. Denkbar sind aber auch anderweitige Federarten, wie beispielsweise Schenkelfedern oder Torsionsfedern. Ferner wäre vorstellbar, das Federelement als ein Elastomer auszubilden.

Nach einem weiteren vorteilhaften Gedanken der Erfindung umfasst die zumindest eine Führungskulisse einen ersten und einen zweiten Kulissenbereich, die durch eine entlang der axialen Richtung (X) des Spritzenkörpers verlaufende fiktive Trennlinie voneinander getrennt sind, wobei der Führungsvorsprung in einer Ausgangsstellung in dem ersten Kulissenbereich anordenbar ist und von dem ersten in den zweiten Kulissenbereich in eine Endstellung durch Überschreiten der Trennlinie überführbar ist, wenn ein distales Ende des Stechelements bei der Relativbewegung von Spritzenkörper zu Hülsenelement auf der Höhe der distalen Öffnung des Hülsenelements angeordnet ist.

Demnach ist der Führungsvorsprung des Kragenelements von dem ersten Kulissenbereich in den zweiten Kulissenbereich überführbar. Diese Überführung findet statt, wenn der Führungsvorsprung eine fiktive Trennlinie, welche den ersten und den zweiten Kulissenbereich voneinander trennt, überschreitet. Befindet sich der Führungsvorsprung in dem ersten Kulissenbereich, also in einer Ausgangsstellung, so wurde die Spritze noch nicht ausgelöst, d.h. das Stechmittel hat die Sicherheitsvorrichtung noch nicht verlassen. Befindet sich der Führungsvorsprung in dem zweiten Kulissenbereich, so ist das Stechmittel aus der Sicherheitsvorrichtung bereits ausgetreten, so dass eine Injektion möglich ist. Beim Übergang von dem ersten Kulissenbereich zu dem zweiten Kulissenbereich, also genau dann, wenn der Führungsvorsprung die Trennlinie überschreitet, befindet sich das distale Ende des Stechmittels auf der Höhe der distalen Öffnung der Sicherheitsvorrichtung.

Bevorzugt ist der Führungsvorsprung von dem zweiten Kulissenbereich mittels einer Kulisse des zweiten Kulissenbereichs in einen Endbereich überführbar, in welchem eine Relativbewegung des Hülsenelements zu dem Spritzenkörper im Wesentlichen entlang der axialen Richtung (X) zumindest eingeschränkt ist. Durch eine derartige Ausgestaltung ist ein weiteres Verschieben des Hülsenelements relativ zum Spritzenkörper zumindest eingeschränkt, bevorzugt verhindert. Demzufolge ist ein weiteres Austreten des Stechmittels aus der Sicherheitsvorrichtung nach der Anwendung der Spritze unterbunden.

Weitere Vorteile, Ziele und Eigenschaften der vorliegenden Erfindung werden anhand nachfolgender Beschreibung der anliegenden Figuren erläutert. Gleichartige Komponenten können in den verschiedenen Ausführungsformen gleiche Bezugszeichen aufweisen.

In den Figuren zeigen:
- Fig.1: eine Schnittdarstellung einer Spritze mit Sicherheitsvorrichtung gemäß einer Ausführungsform;
- Fig.2: eine Schnittdarstellung einer Spritze mit Sicherheitsvorrichtung gemäß einer weiteren Ausführungsform;
- Fig.3: eine Schnittdarstellung einer Spritze mit Sicherheitsvorrichtung gemäß einer weiteren Ausführungsform;
- Fig.4: eine Schnittdarstellung einer Spritze mit Sicherheitsvorrichtung gemäß einer weiteren Ausführungsform;
- Fig.5: eine isometrische Ansicht eines Hülsenelements gemäß einer weiteren Ausführungsform;
- Fig.6: eine isometrische Ansicht eines Hülsenelements gemäß einer weiteren Ausführungsform;
- Fig.7: eine isometrische Ansicht eines Kappenelements gemäß einer weiteren Ausführungsform;
- Fig.8: eine Seitenansicht einer an einem Spritzenkörper angeordneten Sicherheitsvorrichtung.

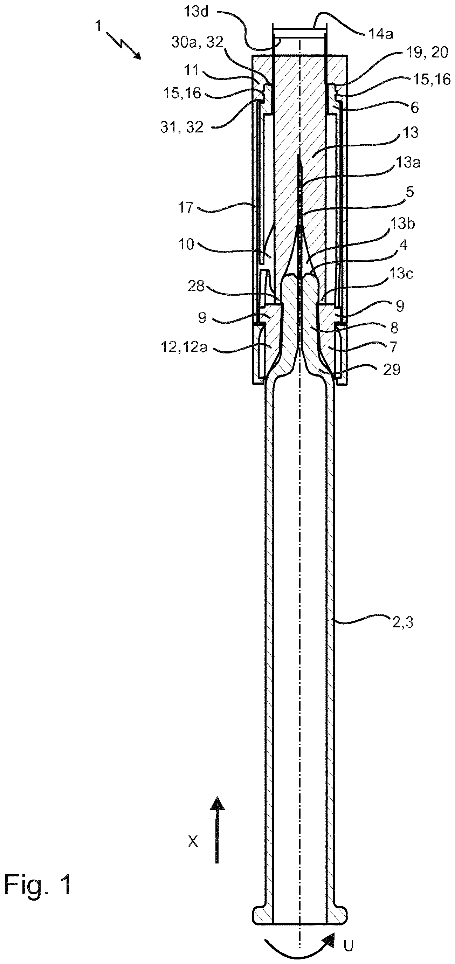

In den Figuren 1 bis 4 ist eine Spritze (2) mit einer Sicherheitsvorrichtung (1) zur Vermeidung von Stichverletzungen gemäß unterschiedlicher Ausführungsformen gezeigt. Die Spritze (2) umfasst einen Spritzenkörper (3), welcher als hohler Kreiszylinder ausgestaltet ist. Der Spritzenkörper weist einen distalen Endbereich (8) mit einem distalen Ende (4) auf. An dem distalen Ende (4) ist ein Stechmittel (5) angeordnet. Dieses Stechmittel (5) ist über eine Bohrung in dem distalen Endbereich (8) mit dem Hohlraum des Spritzenkörpers (3) verbunden, so dass das zu injizierende Medium bei einer Anwendung der Spritze (2) aus dem Hohlraum durch das Stechmittel (5) treten kann. Der distale Endbereich (8) ist als konisches Endstück ausgestaltet, welches einen kleineren Außendurchmesser als der Spritzenkörper (3) aufweist. Weiterhin weist die Spritze einen Übergangsbereich (29) auf, in dem der Außendurchmesser des Spritzenkörpers in den Außendurchmesser des Endstücks übergeht. Darüber hinaus ist an dem distalen Endbereich ein Vorsprung (28) angeordnet.

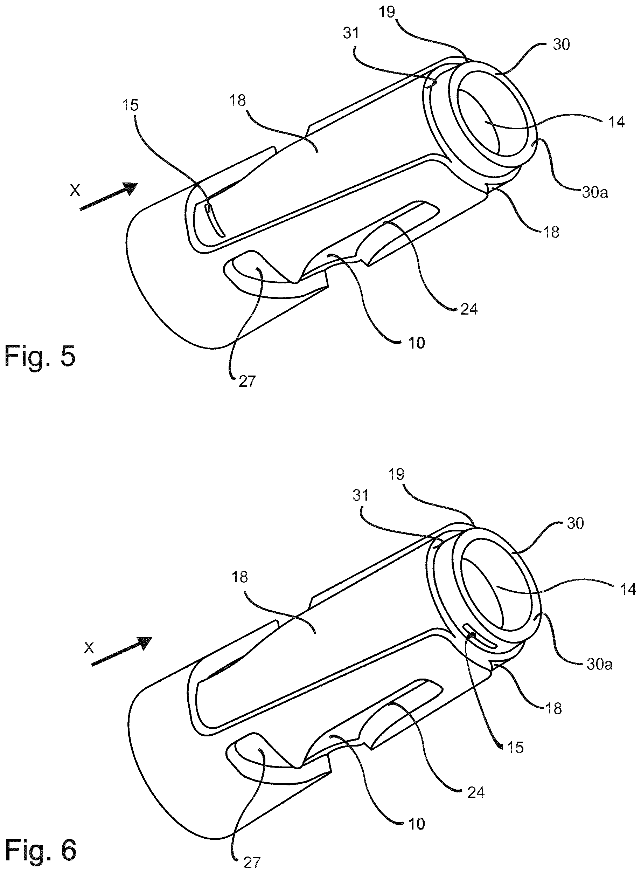

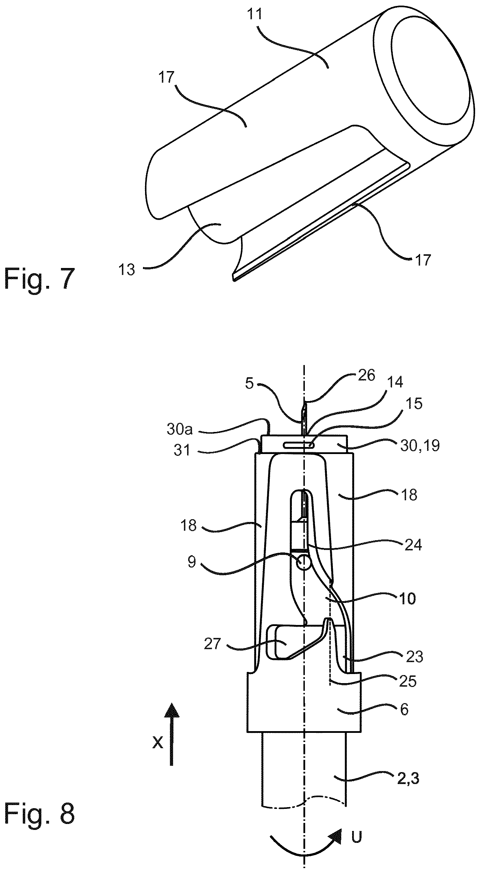

Weiterhin ist eine Sicherheitsvorrichtung (1) zur Vermeidung von Stichverletzungen für eine Spritze (2) gezeigt, welche einen Spritzenkörper (3) und ein an dem distalen Ende (4) des Spritzenkörpers (3) angeordnetes Stechmittel (5) umfasst. Die Sicherheitsvorrichtung (1) umfasst ein sich entlang einer axialen Richtung (X) erstreckendes Hülsenelement (6), welches zumindest teilweise das Stechmittel (5) und den Spritzenkörper (3) umschließt, und ein Kragenelement (7), welches an einem distalen Endbereich (8) des Spritzenkörpers (3) angeordnet ist und die Sicherheitsvorrichtung (1) in axialer Richtung (X) arretiert. Ferner weist die Sicherheitsvorrichtung (1) ein Kappenelement (11) auf, welches zumindest abschnittsweise über dem Hülsenelement (6) anordenbar ist und durch welches das Hülsenelement (6) bezüglich der Relativbewegung des Spritzenkörpers (3) zu dem Hülsenelement (6) arretierbar ist. Das Hülsenelement (6) und das Kappenelement (11) sind dabei im Wesentlichen zylindrisch ausgestaltet. Das Hülsenelement (6) wird weiterhin in den Figuren 5 und 6 detaillier in einer isometrischen Ansicht dargestellt. In Fig. 8 ist eine Seitenansicht des Hülsenelements (6) gezeigt, wobei dieses an dem Spritzenkörper (3) angeordnet ist. Das Kappenelement (11) ist in Fig. 7 detailliert in einer isometrischen Ansicht dargestellt.

Die Arretierung in axialer Richtung wird durch einen Vorsprung (28) bzw. eine Verdickung an dem distalen Ende (4) des Spritzenkörpers ermöglicht, an welchem das Kragenelement (7) mit seinem distalen Ende anliegt.

Das Kragenelement (7) ist im Wesentlichen als hohler Kreiszylinder (12) ausgebildet. Der Kreiszylinder (12) weist eine Mantelfläche (12a) auf, an der zwei Führungsvorsprünge (9) angeordnet sind. Die Führungsvorsprünge (9) erstrecken sich radial von der Mantelfläche (12a) nach außen weg und sind diametral gegenüberliegend angeordnet. Weiterhin sind diese als Kreiszylinder beziehungsweise als Stift ausgebildet. Diese beiden Führungsvorsprünge (9) sind jeweils in einer Führungskulisse (10) des Hülsenelements (6) bei einer Relativbewegung des Spritzenkörpers (3) zu dem Hülsenelement (6) im Wesentlichen entlang der axialen Richtung (X) geführt. Ferner ist das Kragenelement (7) in einer Umfangsrichtung (U) rotierbar an dem distalen Endbereich (8) des Spritzenkörpers (3) angeordnet.

Das Kappenelement (11) weist eine Stechmittelschutzeinrichtung (13) auf, in welcher das Stechmittel (5) anordenbar ist. Darüber hinaus umfasst das Hülsenelement (6) eine distale Öffnung (14), wobei der Innendurchmesser (14a) dieser distalen Öffnung (14) größer ist als der Außendurchmesser (13d) der Stechmittelschutzeinrichtung (13), so dass die Stechmittelschutzeinrichtung (13) innerhalb des Hülsenelements (6) anordenbar ist.

Das distale Ende (26) und ein anschließender distaler Bereich des Stechmittels (5) sind dabei in einem Hohlraum der Stechmittelschutzeinrichtung (13) angeordnet. Der Hohlraum umfasst einen ersten Bereich (13a), wobei die Stechmittelschutzeinrichtung (13) in diesem ersten Bereich an dessen Innenwänden anliegend ist. In einem dritten Bereich (13c) der Stechmittelschutzeinrichtung (13) erstreckt sich der Hohlraum weiterhin über das distale Ende (4) des Spritzenkörpers. Zwischen dem ersten (13a) und dem dritten Bereich (13c) befindet sich ein zweiter Bereich (13b), in dem sich der Innendurchmesser des Hohlraums ausgehend von dem ersten Bereich (13a) bis zu dem dritten Bereich (13c) vergrößert. Die Stechmittelschutzeinrichtung (13) erstreckt sich über den distalen Endbereich (8) des Spritzenkörpers (3) bis zu dem Kragenelement (7). Die Stechmittelschutzeinrichtung (13) ist mit dem Kragenelement (7) in Wirkkontakt, wodurch das Kragenelement (7) bezüglich einer Rotation arretierbar ist.

Das Kappenelement (11) umfasst weiterhin zwei diametral gegenüberliegende flügelartige Elemente (17). Dies ist auch in Fig. 7 zu erkennen. Diese flügelartigen Elemente (17) sind komplementär zu Aufnahmen (18) des Hülsenelements (6) ausgestaltet. Die Aufnahmen sind in Form von Ausnehmungen in dem Hülsenelement (6) ausgestaltet. Dies ist auch in den Figuren 5 und 6 zu erkennen. Wird das Kappenelement (11) auf dem Hülsenelement (6) angeordnet, so sind die beiden flügelartigen Elemente (17) in den beiden Aufnahmen (18) des Hülsenelements (6) aufnehmbar. Die Stechmittelschutzeinrichtung (13) erstreckt sich dabei durch die distale Öffnung (14) des Hülsenelements (6). Denkbar wäre auch, dass zumindest ein flügelartiges Element (17) mit dem Kragenelement (7) in Wirkkontakt ist, wodurch das Kragenelement (7) bezüglich einer Rotation arretierbar ist.

Der distalen Bereich (19) des Hülsenelements (6) umfasst die distale Öffnung (14) des Hülsenelements (6) und einen Kreisring (30), welcher die distale Öffnung (14) des Hülsenelements (6) umrandet. Der Außendurchmesser des Kreisrings (30) ist dabei kleiner als der Außendurchmesser des anschließenden Bereichs des Hülsenelements (6). Somit sind an dem Hülsenelement (6) eine erste (30a) und eine zweite Stirnfläche (31) ausgebildet. Das Kappenelement (11) ist dementsprechend komplementär ausgebildet, so dass dieses in seinem distalen Bereich (20) innenseitig Auflageflächen (32) aufweist, welche an den beiden Stirnflächen (30a, 31) anliegen.

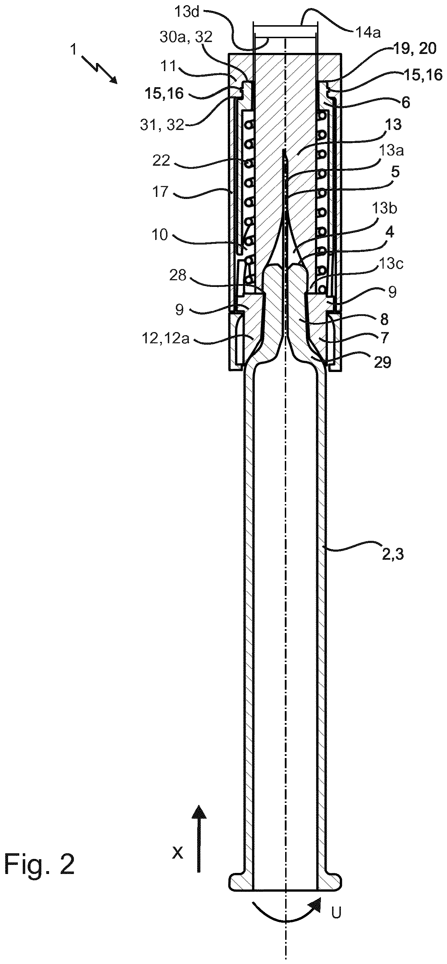

In den Figuren 1 und 2 sind Ausführungsformen der Sicherheitsvorrichtung (1) gezeigt, in denen das Kappenelement (11) integral mit der Stechmittelschutzeinrichtung (13) ausgebildet ist.

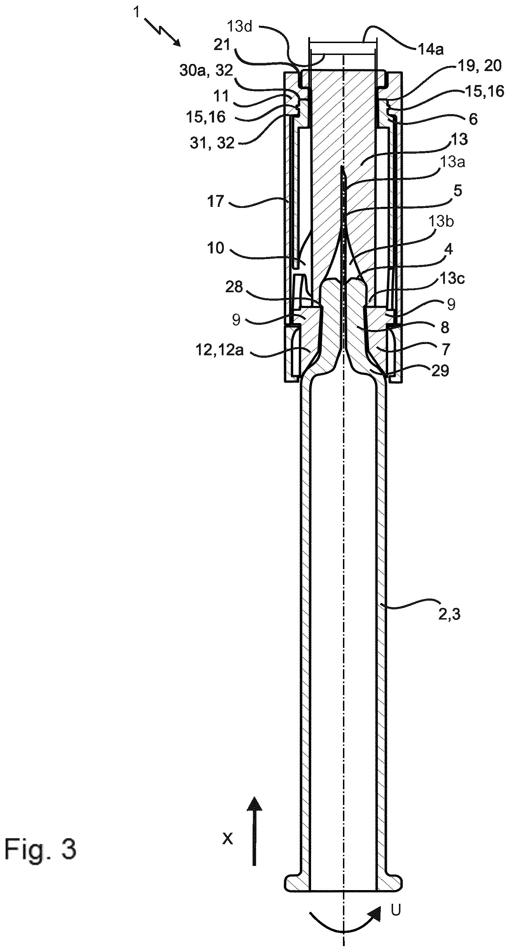

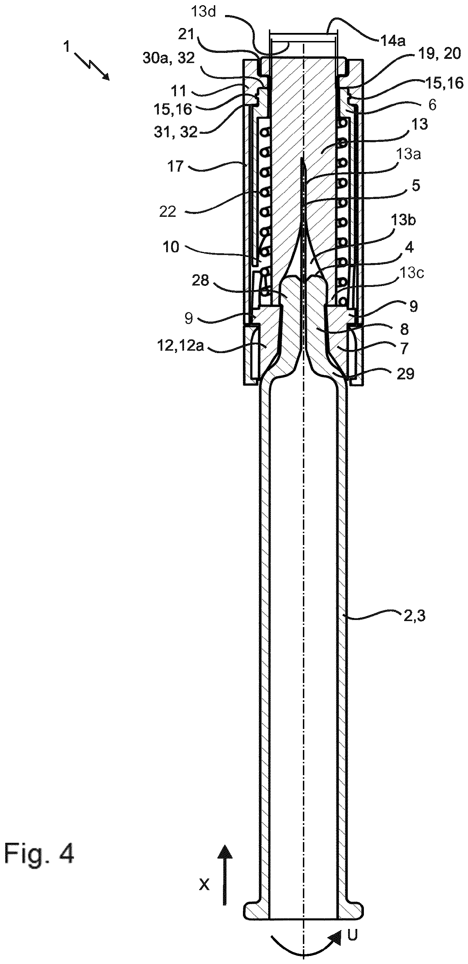

In den Figuren 3 und 4 sind Ausführungsformen der Sicherheitsvorrichtung (1) gezeigt, in denen das Kappenelement (11) eine distale Öffnung (21) aufweist. Die distale Öffnung (21) ist dabei als Aufnahme ausgebildet, um die Stechmittelschutzeinrichtung (13) aufzunehmen.

Die Stechmittelschutzeinrichtung (13) umfasst dabei einen Flansch an dessen distalen Ende, welcher in der Aufnahme der distalen Öffnung (21) des Kappenelements (11) eingebettet ist.

In Fig. 2 und Fig. 4 ist weiterhin eine Sicherheitsvorrichtung (1) dargestellt, welche ein Federelement (22) in Form einer Spiralfeder aufweist, das mit dem Spritzenkörper (3) wirkverbunden ist und der Relativbewegung des Hülsenelements (6) zu der Sicherheitsvorrichtung (1) entgegenwirkt. Demnach bleibt das Stechmittel (5) bis zur vorgesehenen Anwendung innerhalb des Hülsenelements (6). Bei der Anwendung muss das Hülsenelement (6) gegen die Federkraft verschoben werden, damit das Stechmittel (5) durch die distale Öffnung (14) des Hülsenelements (6) hindurchtreten kann. Nach Gebrauch der Spritze (2) schiebt sich automatisch, angetrieben durch die Federkraft des Federelements (22), das Hülsenelement (6) wieder über das Stechmittel (5). Durch die Führung der Führungsvorsprünge (9) in den Führungskulissen (10) rotiert das Kragenelement (7) in entgegen der Umfangsrichtung (U). Der Anwender ist somit vor Stichverletzungen mit dem gebrauchten und kontaminierten Stechmittel geschützt.

Gemäß den Ausführungsformen, gezeigt in den Fig. 1 bis 4 und 6, weisen das Kappenelement (11) und das Hülsenelement (7) zueinander komplementäre Rastelemente (15, 16) auf, so dass das Kappenelement (11) und das Hülsenelement (7) trennbar verrastbar sind. Bei den vorliegenden Ausführungsformen umfasst das Hülsenelement (6) einen distalen Bereich (19), an welchem Rastelemente (15) angeordnet sind. Diese Rastelemente (15) sind in komplementäre Rastelemente (16) einrastbar, welche in einem distalen Bereich (20) des Kappenelements (11) angeordnet sind. Insbesondere sind die Rastelemente (15) des Hülsenelements (6) an einer Mantelfläche des Kreisrings (30) angeordnet.

In Fig. 5 ist ein Hülsenelement (6) dargestellt, welches zwei Aufnahmen (18) für die flügelartigen Elemente (17) des Kappenelements (11) aufweist. In den Aufnahmen (18) sind Rastelemente (15) angeordnet, welche komplementär zu entsprechenden Rastelementen (16) der flügelartigen Elemente (17) des Kappenelements (11) (hier nicht gezeigt) ausgebildet sind und in diese einrastbar sind.

Die Führungskulissen (10) des Hülsenelements (6) umfassen einen ersten (23) und einen zweiten Kulissenbereich (24), welche durch eine entlang des axialen Richtung (X) des Spritzenkörpers (3) verlaufenden fiktiven Trennlinie (25) voneinander getrennt sind, wobei der Führungsvorsprung (9) in einer Ausgangsstellung in dem ersten Kulissenbereich (23) anordenbar ist und von dem ersten (23) in den zweiten Kulissenbereich (24) in eine Endstellung durch Überschreiten der Trennlinie (25) überführbar ist, wenn ein distales Ende (26) des Stechmittels (5) bei der Relativbewegung von Spritzenkörper (3) zu Hülsenelement (6) auf der Höhe der distalen Öffnung (14) des Hülsenelements (6) angeordnet ist. Dies ist in den Figuren 5, 6 und 8 zu erkennen, wobei in Fig. 8 der Führungsvorsprung (9) die Trennlinie (25) überschritten hat, wodurch das distale Ende (26) des Stechmittels (5) bereits über die distale Öffnung (14) des Hülsenelements (6) hinausragt.

Ferner weist das Hülsenelement (6) einen Endbereich (27) auf. Die Führungsvorsprünge (9) sind dabei von dem zweiten Kulissenbereich (24) mittels einer Kulisse des zweiten Kulissenbereichs (24) in einen Endbereich (27) überführbar. In diesem Endbereich (27) ist eine Relativbewegung des Hülsenelements (6) zu dem Spritzenkörper (3) im Wesentlichen entlang der axialen Richtung (X) zumindest eingeschränkt.

### Bezugszeichenliste

- 1: Sicherheitsvorrichtung
- 2: Spritze
- 3: Spritzenkörper
- 4: distales Ende des Spritzenkörpers
- 5: Stechmittel
- 6: Hülsenelement
- 7: Kragenelement
- 8: distaler Endbereich des Spritzenkörpers
- 9: Führungsvorsprung
- 10: Führungskulisse
- 11: Kappenelement
- 12: hohler Kreiszylinder
- 12a: Mantelfläche des Kreiszylinders
- 13: Stechmittelschutzeinrichtung
- 13a: erster Bereich
- 13b: zweiter Bereich
- 13c: dritter Bereich
- 13d: Außendurchmesser
- 14: distale Öffnung des Hülsenelements
- 14a: Innendurchmesser
- 15: Rastelement
- 16: Rastelement
- 17: flügelartiges Element
- 18: Aufnahme
- 19: distalen Bereich des Hülsenelements
- 20: distalen Bereich des Kappenelements
- 21: distale Öffnung des Kappenelements
- 22: Federelement
- 23: erster Kulissenbereich
- 24: zweiter Kulissenbereich
- 25: Trennlinie
- 26: distales Ende des Stechmittels
- 27: Endbereich
- 28: Vorsprung
- 29: Übergangsbereich
- 30: Kreisring
- 30a: erste Stirnfläche
- 31: zweite Stirnfläche
- 32: Auflageflächen
- X: axiale Richtung
- U: Umfangsrichtung

## Patentansprüche

1. Sicherheitsvorrichtung (1) zur Vermeidung von Stichverletzungen für eine Spritze (2) mit einem Spritzenkörper (3) und einem an dem distalen Ende (4) des Spritzenkörpers (3) angeordneten Stechmittel (5), umfassend ein sich entlang einer axialen Richtung (X) erstreckendes Hülsenelement (6), welches zumindest teilweise das Stechmittel (5) und den Spritzenkörper (3) umschließt, und ein Kragenelement (7), welches an einem distalen Endbereich (8) des Spritzenkörpers (3) anordenbar ist und die Sicherheitsvorrichtung (1) in axialer Richtung (X) arretiert, wobei das Kragenelement (7) zumindest einen Führungsvorsprung (9) aufweist, welcher in zumindest einer Führungskulisse (10) des Hülsenelements (6) eingreift, wobei die Sicherheitsvorrichtung (1) ein Kappenelement (11) aufweist, welches zumindest abschnittsweise über dem Hülsenelement (6) anordenbar ist und durch welches das Hülsenelement bezüglich der Relativbewegung des Spritzenkörpers (3) zu dem Hülsenelement (6) arretierbar ist, wobei das Kragenelement (7) in einer Umfangsrichtung (U) rotierbar an dem distalen Endbereich (8) des Spritzenkörpers (3) angeordnet ist, wobei eine Rotation des Kragenelements (7) durch die Relativbewegung des Spritzenkörpers (3) zu dem Hülsenelement (6) und durch die Führung des Führungsvorsprungs (9) in der Führungskulisse (10) verursacht ist, wobei das Kappenelement (11) eine Stechmittelschutzeinrichtung (13) aufweist, in welcher das Stechmittel (5) anordenbar ist und welche mit dem Kragenelement (7) in Wirkkontakt bringbar ist, wodurch das Kragenelement (7) bezüglich der Rotation arretierbar ist.

2. Sicherheitsvorrichtung (1) nach Anspruch 1,
**dadurch gekennzeichnet, dass**
das Kragenelement (7) im Wesentlichen als hohler Kreiszylinder (12) ausgebildet ist, wobei der Kreiszylinder (12) eine Mantelfläche (12a) aufweist, an der der zumindest eine Führungsvorsprung (9) angeordnet ist,.

3. Sicherheitsvorrichtung (1) nach Anspruch 1 oder 2,
**dadurch gekennzeichnet, dass**
das Hülsenelement (6) eine distale Öffnung (14) aufweist, wobei der Innendurchmesser (14a) der distalen Öffnung (14) zumindest abschnittsweise größer ist als der Außendurchmesser (13a) der Stechmittelschutzeinrichtung (13), so dass die Stechmittelschutzeinrichtung (13) innerhalb des Hülsenelements (6) anordenbar ist.

4. Sicherheitsvorrichtung (1) nach einem der vorhergehenden Ansprüche,
**dadurch gekennzeichnet, dass**
das Kappenelement (11) und das Hülsenelement (7) zueinander komplementäre Rastelemente (15, 16) aufweisen, so dass das Kappenelement (11) und das Hülsenelement (7) trennbar verrastbar sind.

5. Sicherheitsvorrichtung (1) nach einem der vorhergehenden Ansprüche,
**dadurch gekennzeichnet, dass**
das Kappenelement (11) zumindest ein flügelartiges Element (17) umfasst, welches in zumindest einer Aufnahme (18) des Hülsenelements (6) aufnehmbar ist.

6. Sicherheitsvorrichtung (1) nach Anspruch 5,
**dadurch gekennzeichnet, dass**
zumindest ein flügelartiges Element (17) mit dem Kragenelement (7) in Wirkkontakt bringbar ist, wodurch das Kragenelement (7) bezüglich einer Rotation arretierbar ist.

7. Sicherheitsvorrichtung (1) nach Anspruch 5
**dadurch gekennzeichnet, dass**
an dem zumindest einen flügelartigen Element (17) des Kappenelements (11) zumindest ein Rastelement (16) angeordnet ist, welches in zumindest ein komplementäres Rastelement (15), das in der zumindest einen Aufnahme (18) des Hülsenelements (6) angeordnet ist, einrastbar ist.

8. Sicherheitsvorrichtung (1) nach einem der vorhergehenden Ansprüche,
**dadurch gekennzeichnet, dass**
das Hülsenelement (6) einen distalen Bereich (19) aufweist, an welchem zumindest ein Rastelement (15) angeordnet ist, wobei das zumindest eine Rastelement (15) in zumindest ein komplementäres Rastelement (16) einrastbar ist, welches in einem distalen Bereich (20) des Kappenelements (11) angeordnet ist.

9. Sicherheitsvorrichtung (1) nach einem der vorhergehenden Ansprüche,
**dadurch gekennzeichnet, dass**
das Kappenelement (11) integral mit der Stechmittelschutzeinrichtung (13) ausgebildet ist.

10. Sicherheitsvorrichtung (1) nach einem der vorhergehenden Ansprüche,
**dadurch gekennzeichnet, dass**
das Kappenelement (11) eine distale Öffnung (21) aufweist, wobei die distale Öffnung (21) als Aufnahme ausgebildet ist, um die Stechmittelschutzeinrichtung (13) aufzunehmen.

11. Sicherheitsvorrichtung (1) nach einem der vorhergehenden Ansprüche,
**dadurch gekennzeichnet, dass**
die Sicherheitsvorrichtung (1) mindestens ein Federelement (22) aufweist, das mit dem Spritzenkörper (3) wirkverbunden ist und der Relativbewegung des Hülsenelements (6) zu der Sicherheitsvorrichtung (1) entgegenwirkt.

12. Sicherheitsvorrichtung (1) nach einem der vorhergehenden Ansprüche,
**dadurch gekennzeichnet, dass**
die zumindest eine Führungskulisse (10) einen ersten (23) und einen zweiten Kulissenbereich (24) umfasst, welche durch eine entlang des axialen Richtung (X) des Spritzenkörpers (3) verlaufende fiktive Trennlinie (25) voneinander getrennt sind, wobei der Führungsvorsprung (9) in einer Ausgangsstellung in dem ersten Kulissenbereich (23) anordenbar ist und von dem ersten (23) in den zweiten Kulissenbereich (24) in eine Endstellung durch Überschreiten der Trennlinie (25) überführbar ist, wenn ein distales Ende (26) des Stechmittels (5) bei der Relativbewegung von Spritzenkörper (3) zu dem Hülsenelement (6) auf der Höhe der distalen Öffnung (14) des Hülsenelements (6) angeordnet ist.

13. Sicherheitsvorrichtung (1) nach Anspruch 12,
**dadurch gekennzeichnet, dass**
der zumindest eine Führungsvorsprung (9) von dem zweiten Kulissenbereich (24) mittels einer Kulisse des zweiten Kulissenbereichs (24) in einen Endbereich (27) überführbar ist, in welchem eine Relativbewegung des Hülsenelements (6) zu dem Spritzenkörper (3) im Wesentlichen entlang der axialen Richtung (X) zumindest eingeschränkt ist.

## Claims

1. Safety device (1) for preventing piercing wounds for a syringe (2) having a syringe body (3) and a piercing means (5) arranged at the distal end (4) of the syringe body (3), comprising a sleeve element (6) which extends along an axial direction (X) and at least partially encloses the piercing means (5) and the syringe body (3), and a collar element (7) which can be arranged on a distal end region (8) of the syringe body (3) and locks the safety device (1) in the axial direction (X), wherein the collar element (7) comprises at least one guide projection (9) which engages in at least one guide track (10) of the sleeve element (6),
wherein
the safety device (1) comprises a cap element (11) which can be arranged over the sleeve element (6) at least in portions and by means of which the sleeve element can be locked with respect to the movement of the syringe body (3) relative to the sleeve element (6), wherein the collar element (7) is arranged on the distal end region (8) of the syringe body (3) so as to be able to rotate in a circumferential direction (U), wherein a rotation of the collar element (7) is brought about by the movement of the syringe body (3) relative to the sleeve element (6) and by the guide projection (9) being guided in the guide track (10), wherein
the cap element (11) comprises a piercing means protective device (13) in which the piercing means (5) can be arranged and which can be brought into operative contact with the collar element (7), as a result of which the collar element (7) can be locked with respect to the rotation.

2. Safety device (1) according to claim 1,
**characterised in that**
the collar element (7) is substantially formed as a hollow circular cylinder (12), the circular cylinder (12) comprising a lateral surface (12a) on which the at least one guide projection (9) is arranged.

3. Safety device (1) according to either claim 1 or claim 2,
**characterised in that**
the sleeve element (6) comprises a distal opening (14), the internal diameter (14a) of the distal opening (14) being larger than the external diameter (13a) of the piercing means protective device (13) at least in portions, such that the piercing means protective device (13) can be arranged inside the sleeve element (6).

4. Safety device (1) according to any of the preceding claims,
**characterised in that**
the cap element (11) and the sleeve element (7) comprise complementary latching elements (15, 16) so that the cap element (11) and the sleeve element (7) can be latched together in a separable manner.

5. Safety device (1) according to any of the preceding claims,
**characterised in that**
the cap element (11) comprises at least one wing-like element (17) which can be received in at least one receptacle (18) of the sleeve element (6).

6. Safety device (1) according to claim 5,
**characterised in that**
at least one wing-like element (17) can be brought into operative contact with the collar element (7), as a result of which the collar element (7) can be locked with respect to a rotation.

7. Safety device (1) according to claim 5,
**characterised in that**
at least one latching element (16) is arranged on the at least one wing-like element (17) of the cap element (11), which latching element can be latched into at least one complementary latching element (15) that is arranged in the at least one receptacle (18) of the sleeve element (6).

8. Safety device (1) according to any of the preceding claims,
**characterised in that**
the sleeve element (6) comprises a distal region (19) on which at least one latching element (15) is arranged, it being possible for the at least one latching element (15) to be latched into at least one complementary latching element (16) that is arranged in a distal region (20) of the cap element (11).

9. Safety device (1) according to any of the preceding claims,
**characterised in that**
the cap element (11) is formed integrally with the piercing means protective device (13).

10. Safety device (1) according to any of the preceding claims,
**characterised in that**
the cap element (11) comprises a distal opening (21), the distal opening (21) being formed as a receptacle, in order to receive the piercing means protective device (13).

11. Safety device (1) according to any of the preceding claims,
**characterised in that**
the safety device (1) comprises at least one spring element (22) which is operatively connected to the syringe body (3) and counteracts the movement of the sleeve element (6) relative to the safety device (1).

12. Safety device (1) according to any of the preceding claims,
**characterised in that**
the at least one guide track (10) comprises a first (23) and a second track region (24), which are separated from one another by a fictive separating line (25) extending along the axial direction (X) of the syringe body (3), it being possible for the guide projection (9) to be arranged in a starting position in the first track region (23) and to be moved from the first track region (23) into an end position in the second track region (24) by passing the separating line (25) when a distal end (26) of the piercing means (5) is arranged at the level of the distal opening (14) of the sleeve element (6) as the syringe body (3) is moved relative to the sleeve element (6).

13. Safety device (1) according to claim 12,
**characterised in that**
the at least one guide projection (9) can be moved, by means of a slot of the second track region (24), from the second track region (24) into an end region (27) in which a movement of the sleeve element (6) relative to the syringe body (3) is at least limited substantially along the axial direction (X).

## Revendications

1. Dispositif de sécurité (1) pour éviter des blessures par piqûre pour une seringue (2) comportant un corps de seringue (3) et un moyen de perçage (5) disposé à l'extrémité distale (4) du corps de seringue (3), comportant un élément manchon (6) s'étendant le long d'une direction axiale (X), lequel enferme au moins partiellement le moyen de perçage (5) et le corps de seringue (3), et un élément collier (7), lequel est apte à être disposé sur une zone d'extrémité distale (8) du corps de seringue (3) et qui verrouille le dispositif de sécurité (1) en direction axiale (X), l'élément collier (7) présentant au moins une saillie de guidage (9), laquelle vient en prise dans au moins une coulisse de guidage (10) de l'élément manchon (6),
dans lequel
le dispositif de sécurité (1) présente un élément capuchon (11), lequel est apte à être disposé au moins par endroits sur l'élément manchon (6) et au moyen duquel l'élément manchon est apte à être verrouillé en ce qui concerne le déplacement relatif du corps de seringue (3) par rapport à l'élément manchon (6), dans lequel l'élément collier (7) est disposé apte à tourner dans une direction périphérique (U) sur la zone d'extrémité distale (8) du corps de seringue (3), dans lequel une rotation de l'élément collier (7) est provoquée par le déplacement relatif du corps de seringue (3) par rapport à l'élément manchon (6) et par le guidage de la saillie de guidage (9) dans la coulisse de guidage (10), dans lequel
l'élément capuchon (11) présente un dispositif de protection de moyen de perçage (13), dans lequel le moyen de perçage (5) est apte à être disposé et qui est apte à être amené en contact fonctionnel avec l'élément collier (7), ce par quoi l'élément collier (7) est apte à être verrouillé en ce qui concerne la rotation.

2. Dispositif de sécurité (1) selon la revendication 1,
**caractérisé par le fait que**
l'élément collier (7) est réalisé essentiellement en tant que cylindre à base circulaire creux (12), le cylindre à base circulaire (12) présentant une surface d'enveloppe (12a), sur laquelle ladite au moins une saillie de guidage (9) est disposée.

3. Dispositif de sécurité (1) selon l'une des revendications 1 ou 2,
**caractérisé par le fait que**
l'élément manchon (6) présente une ouverture distale (14), le diamètre interne (14a) de l'ouverture distale (14) étant au moins par endroits plus grand que le diamètre externe (13a) du dispositif de protection de moyen de perçage (13), de telle sorte que le dispositif de protection de moyen de perçage (13) est apte à être disposé à l'intérieur de l'élément manchon (6).

4. Dispositif de sécurité (1) selon l'une des revendications précédentes,
**caractérisé par le fait que**
l'élément capuchon (11) et l'élément manchon (7) présentent des éléments d'encliquetage (15, 16) complémentaires l'un par rapport à l'autre, de telle sorte que l'élément capuchon (11) et l'élément manchon (6) sont encliquetables de façon séparable.

5. Dispositif de sécurité (1) selon l'une des revendications précédentes,
**caractérisé par le fait que**
l'élément capuchon (11) comporte au moins un élément (17) en forme d'aile, lequel est apte à être reçu dans au moins un logement (18) de l'élément manchon (6).

6. Dispositif de sécurité (1) selon la revendication 5,
**caractérisé par le fait que**
au moins un élément (17) en forme d'aile est apte à être amené en contact fonctionnel avec l'élément collier (7), ce par quoi l'élément collier (7) est apte à être verrouillé en ce qui concerne une rotation.

7. Dispositif de sécurité (1) selon la revendication 5,
**caractérisé par le fait que**
sur ledit au moins un élément (17) en forme d'aile de l'élément capuchon (11), au moins un élément d'encliquetage (16) est disposé, lequel est apte à s'encliqueter dans au moins un élément d'encliquetage complémentaire (15), lequel est disposé dans ledit au moins un logement (18) de l'élément manchon (6).

8. Dispositif de sécurité (1) selon l'une des revendications précédentes,
**caractérisé par le fait que**
l'élément manchon (6) présente une zone distale (19), sur laquelle au moins un élément d'encliquetage (15) est disposé, ledit au moins un élément d'encliquetage (15) étant apte à s'encliqueter dans au moins un élément d'encliquetage complémentaire (16), lequel est disposé dans une zone distale (20) de l'élément capuchon (11).

9. Dispositif de sécurité (1) selon l'une des revendications précédentes,
**caractérisé par le fait que**
l'élément capuchon (11) est conçu d'un seul tenant avec le dispositif de protection de moyen de perçage (13).

10. Dispositif de sécurité (1) selon l'une des revendications précédentes,
**caractérisé par le fait que**
l'élément capuchon (11) présente une ouverture distale (21), l'ouverture distale (21) étant formée en tant que logement, afin de recevoir le dispositif de protection de moyen de perçage (13).

11. Dispositif de sécurité (1) selon l'une des revendications précédentes,
**caractérisé par le fait que**
le dispositif de sécurité (1) présente au moins un élément ressort (22), lequel est relié de façon fonctionnelle avec le corps de seringue (3) et s'oppose au déplacement relatif de l'élément manchon (6) par rapport au dispositif de sécurité (1).

12. Dispositif de sécurité (1) selon l'une des revendications précédentes,
**caractérisé par le fait que**
ladite au moins une coulisse de guidage (10) comporte une première (23) et une seconde (24) zone de coulisse, lesquelles sont séparées l'une de l'autre par une ligne de séparation fictive (25) s'étendant le long de la direction axiale (X) du corps de seringue (3), la saillie de guidage (9) étant apte à être disposée dans une position de départ dans la première zone de coulisse (23) et étant apte à être amenée de la première zone de coulisse (23) dans la seconde zone de coulisse (24) dans une position finale par dépassement de la ligne de séparation (25), lorsqu'une extrémité distale (26) du moyen de perçage (5) est disposée au niveau de l'ouverture distale (14) de l'élément manchon (6) lors du déplacement relatif du corps de seringue (3) par rapport à l'élément manchon (6).

13. Dispositif de sécurité (1) selon la revendication 12,
**caractérisé par le fait que**
ladite au moins une saillie de guidage (9) est apte à être amenée, au moyen d'une coulisse de la seconde zone de coulisse (24), de la seconde zone de coulisse (24) dans une zone d'extrémité (27), dans laquelle un déplacement relatif de l'élément manchon (6) par rapport au corps de seringue (3) est au moins limité, essentiellement le long de la direction axiale (X).
